# EUROPEAN PATENT APPLICATION

(11) **EP 2 128 162 A1**
(43) Date of publication of application: **02.12.2009**
(21) Application number: 08704041.6
(22) Date of filing: 29.01.2008
(51) Int. Cl.: C07D 499/68, A61K 9/14, B01D 9/00, B01D 9/02

(54) **NOVEL CRYSTAL OF PIPERACILLIN SODIUM**

(30) Priority: 31.01.2007 JP 2007020758
(71) Applicant: Toyama Chemical Co., Ltd., Shinjuku-ku Tokyo 160-0023 (JP)
(72) Inventor: MOURI, Masaru, Toyama-shi Toyama 930-8508 (JP); NISHIKAWA, Dai, Toyama-shi Toyama 930-8508 (JP)
(74) Representative: Hartz, Nikolai
(86) International application number: PCT/JP2008/051232
(87) International publication number: WO 2008/093650

(57) **Abstract**

Disclosed are a novel crystal of piperacillin sodium which has diffraction angles of 3.7°, 5.5°, 7.3°, 11.6°, 14.5° and 18.0° and a novel crystal of piperacillin sodium which has diffraction angles of 5.6°, 7.8°, 12.3°, 15.5°, 17.5°, 23.3°, 24.8° and 28.5° expressed by 2θ in the powder X-ray diffraction analysis. The crystals have excellent stability and solubility and a low water-absorbing property, is extremely easy to be handled in the manufacturing process for a medicinal substance and in the packing during the preparation process for a pharmaceutical product. A pharmaceutical preparation for injection comprising any one of the crystals is useful.

## Description

### FIELD OF THE INVENTION

The present invention relates to novel crystals of (2S,5R,6R)-6-((2R)-2-((4-ethyl-2,3-dioxopiperazine-1-carbonyl)amino)-2-phenylacetylamino)-3,3-dimethyl-7-oxo-4-thia-1-azabicyclo[3.2.0]heptane-2-carboxylic acid (hereinafter called as "piperacillin") sodium.

### BACKGROUND ART

It is known that piperacillin or the salt thereof has wide antimicrobial activity and takes effect against, especially Gram negative microorganism, Pseudomonas aeruginosa, Proteus vulgaris, Serratia species, bowel bacteria and other anaerobes which are clinically important.
For example, the sodium salt of piperacillin is used effectively to the treatment for pneumonitis, purulent meningitis, sepsis and the like.
In general, as injections of drugs, it is known that there are liquid injections which are used by being dissolved, emulsified and dispersed in liquids including water and the like, and it is known that there are timely soluble powder injections which are used by being dissolved, emulsified and dispersed in liquids when these are used.
However, piperacillin or the salt thereof is unstable in solution, and it is difficult to manufacture liquid injections of piperacillin or the salt thereof.
In addition, piperacillin or the salt thereof is obtained as solids of amorphous body till now, and therefore freeze-dried powders, for example, had been used for powder-packing pharmaceutical preparations of piperacillin.
However, amorphous body is hygroscopic, and is inferior in timely solubility.
Furthermore, the manufacture by freeze-drying needs long time, and not only raises manufacturing costs but also had a problem that the purification was difficult.
There is the description that the salt of piperacillin was obtained as crystals in bulletins such as Japanese Patent Laid-Open No. H02-32082 (Patent document 1) and the publication of the United States patent application No. 2003/028016 (Patent document 2) and the like, but there are no data which suggest that they are crystalline at all.
Although the inventors of the present application carried out supplementary examinations of example of the above documents, the crystal of piperacillin was not obtained.
The crystals of the salt of piperacillin as disclosed in the present application have not been known at all till now.

[Patent document 1] Japanese Patent Laid-Open No. H02-32082 bulletin
[Patent document 2] The publication of the United States patent application No. 2003/028016 specification
[Patent document 3] Japanese Patent Laid-Open No. S51-23284 bulletin

### DISCLOSURE OF THE INVENTION

### PROBLEM TO BE SOLVED BY THE INVENTION

The present invention is to provide novel crystals of piperacillin sodium having excellent actions as a medicine and to provide injectable pharmaceutical preparations which are filled up with the crystals.

### MEANS TO SOLVE THE PROBLEMS

The present inventors have found that there is crystalline body of piperacillin sodium and found that crystalline polymorphism exists in the crystalline body as a result of having repeated researches zealously to solve the above problems, and have completed the present invention.
The present invention is explained below in detail.

### EFFECTS OF THE INVENTION

The novel crystals of piperacillin sodium of the present invention have superior solubility, and the handling of those is very easy in the case of the manufacture of a bulk drug and in the case of the filling at formulation because of low hygroscopicity.
In addition, these crystals are easily filtrated and dried, have small content of relative substances, are high purity and are suitable for a medicinal bulk drug.
Furthermore, these crystals are superior in stability, and the purity depressions and the colorations (degrees of whiteness and yellowness) of exterior appearance of these crystals are smaller than those of the amorphous body.
For these points, the novel crystals of piperacillin sodium of the present invention and the injectable pharmaceutical preparations which were filled up with these crystals are useful.

### BEST MODE FOR CARRYING OUT THE INVENTION

In the present invention, as far as it is not mentioned in particular, supercritical carbon dioxide means carbon dioxide of supercritical state where the critical temperature of carbon dioxide is equal to or more than 31.1°C and the critical pressure of carbon dioxide is equal to or more than 7.38 MPa; alkylketones mean, for example, acetone, 2-butanone and methyl isobutyl ketone; alkyl esters mean, for example, methyl acetate, ethyl acetate and butyl acetate; alcohols mean, for example, methanol, ethanol, propanol, 2-propanol, butanol, t-butanol, ethylene glycol and propylene glycol; ethers mean, for example, tetrahydrofuran, 1,4-dioxane, dimethylether, diethylether, diisopropylether, t-butyl methyl ether and 1,2-dimethoxyethane.

The present invention relates to the crystals of piperacillin sodium monohydrate (hereinafter called as "V type crystal") having diffraction angles of 3.7, 5.5, 7.3, 11.6, 14.5 and 18.0° expressed by 2θ in the powder X-ray diffraction analysis and the crystal of piperacillin sodium monohydrate (hereinafter called as "VI type crystal") having diffraction angles of 5.6, 7.8, 12.3, 15.5, 17.5, 23.3, 24.8 and 28.5° expressed by 2θ in the powder X-ray diffraction analysis. These are the novel crystals which were not known till now. Further, characteristic peaks in the powder X-ray diffraction analysis may vary with measurement conditions as well. Therefore, the peaks of the powder X-ray diffraction analysis of the compound of the present invention are not interpreted strictly.

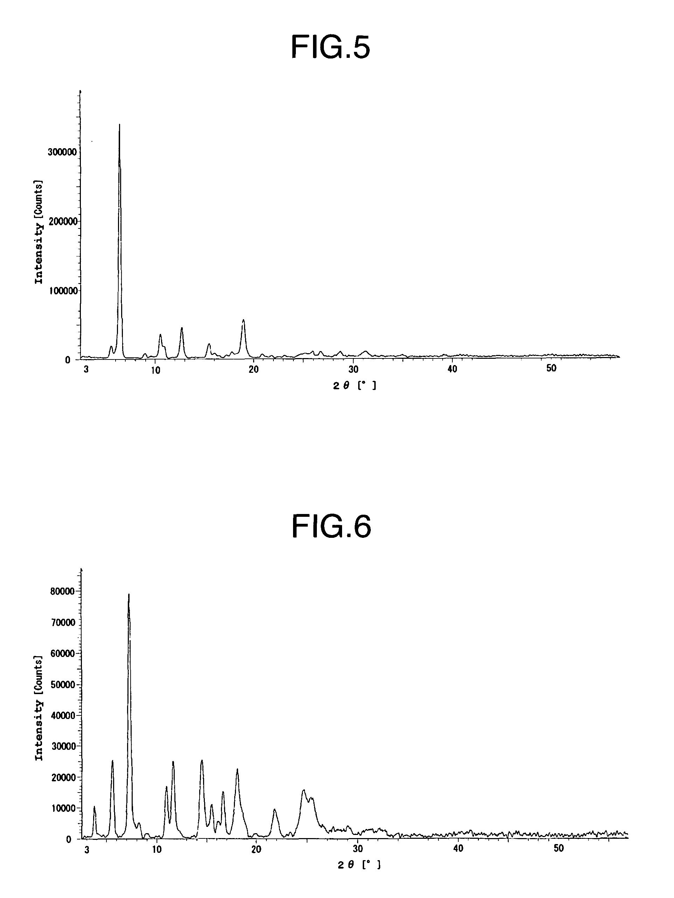

Figures 6 to 8 show the result of the measurement of the powder X-ray diffraction analysis of the V type crystal, the VI type crystal and freeze-dried piperacillin sodium (hereinafter called as "amorphous body").

A powder X-ray diffraction measurement conditions
X-ray used: CuKα
X-ray detector: 2-dimensional detector
Applied voltage: 40 kV
Applied current: 40 mA
Goniometer: sample flat stage-type (2 axis)
Sample stage: 5-axis sample stage
Collimator diameter: 300 µmφ
Scan axis: 2θ
Scanning field: 2θ=2.5 to 57°
Distance of between sample and detector: 25 cm
Measuring time: 180 seconds

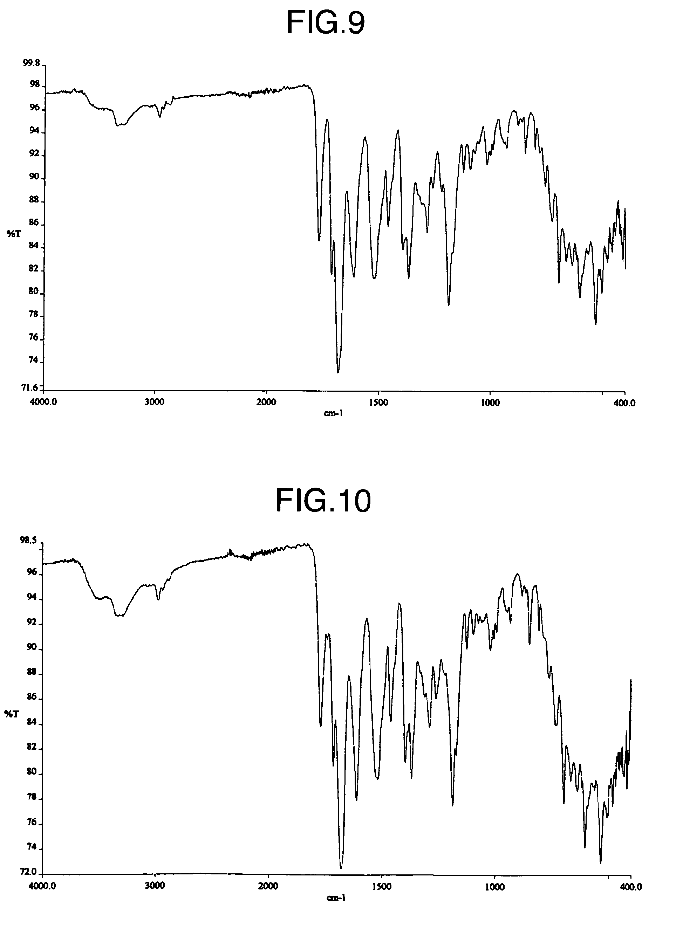

The V type crystal and the VI type crystal of the present invention are the novel crystals which are characterized as well by the absorption wave length (cm⁻¹) of infrared absorption spectrum measured in the following conditions. Figures 10 to 12 show the result of measurement of the infrared absorption spectrum of the V type crystal, the VI type crystal and the amorphous body.

### Infrared absorption spectrum measurement conditions

It was measured according to Japanese pharmacopoeia, general tests, infrared absorption spectrum attenuated total reflection method (ATR method).

Next, the results of dissolution tests, hygroscopic tests, purity tests, purity stability tests, coloration stability tests and light exposure stability tests are explained to elucidate the utility of the compounds of the present invention.

### (1) Dissolution test

As test substances, the V type crystal (Example 1), the VI type crystal (Example 2) and the amorphous body (Comparative example 1) were used.
Test substance 2.2 g was put in a C2 vial made by Daiichi Glass Co., Ltd., injectable water 10 mL was added to the vial, the vial was fixed laterally and was shaken by MRK incubator-shaker (4cm of shaking width, 48 times/minute of shaking rate).
Complete dissolution was judged by an unaided eye, and the length of time to need for the complete dissolution was measured.
The test was carried out at three times respectively, and those mean values were assumed to the dissolution time.
The results are shown in Table 1.

**Table 1**

| **Test substance** | **Dissolution time** |
|---|---|
| **Comparative example 1** | **5 minutes and 10 seconds** |
| **Example 1** | **43 seconds** |
| **Example 2** | **50 seconds** |

The dissolution time of the substance of Comparative example 1 was 5 minutes and 10 seconds. On the other hand, the dissolution time of the substances of Example 1 and Example 2 were 50 seconds and 43 seconds respectively.
The V type crystal and the VI type crystal dissolved in a short period of time than the amorphous body.

### (2) Hygroscopic test

As test substances, the V type crystal (Example 1), the VI type crystal (Example 2) and the amorphous body (Comparative example 1) were used.
Test substances were allowed to stand at 25°C for three days under 7, 11, 22, 33 and 57% relative humidity, the content of moisture was measured according to Japanese pharmacopoeia, general tests, water determination method (Karl Fischer's method) and the changes of weight were calculated.
The results are shown in Table 2.

**Table 2**

| **Relative humidity (RH%)** | **Ratio of change of weight (w/w%)** | | |
|---|---|---|---|
| | **Comparative example 1** | **Example 1** | **Example 2** |
| **7** | **2.20** | **0** | **1.16** |
| **11** | **3.12** | **0.93** | **1.54** |
| **22** | **4.99** | **3.18** | **3.14** |
| **33** | **6.78** | **4.77** | **3.98** |
| **57** | **11.03** | **8.43** | **5.28** |

The substances of Example 1 and Example 2 had the smaller ratio of change of weight than the substance of Comparative example 1.
The V type crystal and the VI type crystal were superior in the moisture absorption stability to the amorphous body.

### (3) Purity (the content of relative substances) test

As test substances, the V type crystal (Example 1), the VI type crystal (Example 2) and the amorphous body (Comparative example 1) were used.
Purity of the test substances was measured by liquid chromatography method (a notice of the 340th April 4, 2001 by Ministry of Health and Welfare medicinal safe directors of bureaus).

By-product I and decomposition product I measurement conditions
Detector: ultraviolet absorption photometer
Measurement wavelength: 254nm
Column: Develosil ODS-HG-5, 4.6 mm inside diameter and 150 mm length
Column temperature: room temperature
Mobile phase: 21% acetonitrile buffer solution (0.05 mol/L acetic acid, 0.025 mol/L triethylamine)
Flow rate: 1 ml/minute

By-product II measurement conditions
Detector: ultraviolet absorption photometer
Measurement wavelength: 254nm
Column: Nucleosil-5C18, 4.6 mm inside diameter and 150 mm length
Column temperature: room temperature
Mobile phase: 30% acetonitrile buffer solution (0.05 mol/L acetic acid, 0.025 mol/L triethylamine)
Flow rate: 1 ml/minute

The results are shown in Table 3.

**[Table 3]**

| **Test substance** | **The content of relative substances(%)** | | |
|---|---|---|---|
| | **By-product I** | **Decomposition product I** | **By-product II** |
| **Comparative example 1** | **0.22** | **0.01** | **0.22** |
| **Example 1** | **0.04** | **Not detected** | **Not detected** |
| **Example 2** | **0.02** | **Not detected** | **Not detected** |

The contents of relative substances of the substance of Example 1 and Example 2 were smaller than those of the substance of Comparative example 1.

### (4) Purity stability test

As test substances, the V type crystal (Example 1), the VI type crystal (Example 2) and the amorphous body (Comparative example 1) were used.
Test substance 2.2 g was put in a C2 vial made by Daiichi Glass Co., Ltd., and after the vial was filled with nitrogen gas, the vial was stored at 60°C. After the initiation of the test, the purity of each test substances was measured by high-performance liquid chromatographic method at 1 week, 2 weeks and 4 weeks later (a notice of the 340th April 4, 2001 by Ministry of Health and Welfare medicinal safe directors of bureaus).
The results are shown in Table 4.

**[Table 4]**

| **Test substance** | **Test condition** | **By-product I** | **Decomposition product I** | **Piperacillin** |
|---|---|---|---|---|
| **Comparative example 1** | **Initiation of test** | **0.22** | **0.01** | **99.51** |
| | **60°C, 1 week** | **0.80** | **0.13** | **98.27** |
| | **60°C, 2 weeks** | **0.97** | **0.17** | **98.05** |
| | **60°C, 4 weeks** | **1.21** | **0.30** | **97.04** |
| **Example 1** | **Initiation of test** | **0.04** | **Not detected** | **99.92** |
| | **60°C, 1 week** | **0.21** | **0.03** | **99.66** |
| | **60°C, 2 weeks** | **0.33** | **0.08** | **99.37** |
| | **60°C, 4 weeks** | **0.59** | **0.26** | **98.62** |
| **Example 2** | **Initiation of test** | **0.02** | **Not detected** | **99.94** |
| | **60°C, 1 week** | **0.07** | **0.01** | **99.82** |
| | **60°C, 2 weeks** | **0.11** | **0.02** | **99.75** |
| | **60°C, 4 weeks** | **0.11** | **0.04** | **99.73** |

The increments of the content of the by-product I and the decomposition product I of the substances of Example 1 and Example 2 were smaller than those of the substance of Comparative example 1, and the depressions of the content of piperacillin of the substances of Example 1 and Example 2 were smaller that those of the substance of Comparative example 1. The depressions of purity of the V type crystal and the VI type crystal were smaller than those of the amorphous body, and the V type crystal and the VI type crystal were stable.

### (5) Coloration stability test

As test substances, the V type crystal (Example 1), the VI type crystal (Example 2) and the amorphous body (Comparative example 1) were used.
Test substance 2.2 g was put in a C2 vial made by Daiichi Glass Co., Ltd., and after the vial was filled with nitrogen gas, the vial was stored at 60°C. After the initiation of the test, the color difference (ΔE), degree of whiteness (W) and yellowness (YI) of each test substances were measured by a color-difference meter at 1 week, 2 weeks and 4 weeks later.
The results are shown in Table 5.

**[Table 5]**

| **Test substance** | **Test condition** | **Color difference (**Δ**E)** | **Degree of whiteness (W)** | **Degree of yellowness (YI)** |
|---|---|---|---|---|
| **Comparative example 1** | **Initiation of test** | **-** | **96.86** | **2.17** |
| | **60°C, 1 week** | **2.76** | **94.66** | **6.51** |
| | **60°C, 2 weeks** | **4.27** | **93.29** | **8.97** |
| | **60°C, 4 weeks** | **5.73** | **91.87** | **11.25** |
| **Example 1** | **Initiation of test** | **-** | **96.45** | **0.94** |
| | **60°C, 1 week** | **0.60** | **96.64** | **1.77** |
| | **60°C, 2 weeks** | **0.69** | **96.55** | **1.96** |
| | **60°C, 4 weeks** | **1.33** | **96.20** | **3.03** |
| **Example 2** | **Initiation of test** | **-** | **95.94** | **0.85** |
| | **60°C, 1 week** | **0.39** | **95.64** | **1.35** |
| | **60°C, 2 weeks** | **0.54** | **95.60** | **1.64** |
| | **60°C, 4 weeks** | **0.79** | **95.57** | **2.02** |

The color differences of the substances of Example 1 and Example 2 were smaller than those of the substance of Comparative example 1, and the degrees of whiteness of the substances of Example 1 and Example 2 of were greater than those of the substance of Comparative example 1, and the degrees of yellowness of the substances of Example 1 and Example 2 were smaller than those of the substance of Comparative example 1. The color variations of exterior appearance of the V type crystal and the VI type crystal were smaller than those of the amorphous body, and the V type crystal and the VI type crystal were stable.

### (6) Light exposure stability test

As test substances, the VI type crystal (Example 2) and the amorphous body (Comparative example 1) were used.

Test substance 2.2 g was put in a C2 vial made by Daiichi Glass Co., Ltd., and after the vial was filled with nitrogen gas, the vial was radiated (120million lx/hr) with D65 lamp (FLR20S-D-EDL-D65/M) at 25°C.
After the initiation of the test and the radiation of 120million lx/hr, the purity of each test substances was measured by high-performance liquid chromatographic method (a notice of the 340th April 4, 2001 by Ministry of Health and Welfare medicinal safe directors of bureaus).
The results are shown in Table 6.

**[Table 6]**

| **Test substance** | **Test condition** | **By-product I** | **Total amount of relative substances** | **Piperacillin** |
|---|---|---|---|---|
| **Comparative example 1** | **Initiation of test** | **0.23** | **0.90** | **99.10** |
| | **1.20 million lx-hr** | **0.31** | **1.14** | **98.86** |
| **Example 2** | **Initiation of test** | **0.15** | **0.19** | **99.81** |
| | **1.20 million 1x-hr** | **0.14** | **0.27** | **99.73** |

The increments of the content of the by-product I and the total amount of relative substances of the substance of Example 2 were smaller than those of the substance of Comparative example 1, and the depressions of the amount of piperacillin of the substance of Example 2 were smaller that those of the substance of Comparative example 1. The depressions of purity of the VI type crystal were smaller than those of the amorphous body, and the type VI crystal was stable.

Manufacturing methods of the V type crystal and the VI type crystal of the present invention are explained next. The V type crystal and the VI type crystal can be manufactured, for example, in the following manufacturing methods.

### [Manufacturing method 1]

### The manufacture of the V type crystal

The V type crystal can be manufactured by contacting a crystal (hereinafter called as "III type crystal") of piperacillin sodium monohydrate having diffraction angles of 6.1, 10.2, 12.4, 15.1, 15.8 and 18.6° expressed by 2θ in the powder X-ray diffraction analysis with supercritical carbon dioxide in the presence of solvent, subsequently by contacting with supercritical carbon dioxide.
The III type crystal, which is an important manufacturing intermediate of the V type crystal, can be manufactured by the after-mentioned manufacturing method A.
For solvents used in this manufacture, at least one kind of solvent selected from alkylketones, alkyl esters and alcohols is given.
For alkylketones, acetone, 2-butanone and methyl isobutyl ketone are desirable, and acetone and 2-butanone are preferable.
For alkyl esters, methyl acetate and ethyl acetate are desirable, and methyl acetate is preferable.
For alcohols, methanol, ethanol, propanol and 2-propanol are desirable, and ethanol, propanol and 2-propanol are preferable.
In the manufacturing process, the solvent is mixed with supercritical carbon dioxide and used as fluid mixture.
The mixing ratio of solvent and supercritical carbon dioxide is desirably 0.1 to 10 (w/w)% of the ratio of the content of solvent, and is preferably 1 to 5 (w/w)%.

In this manufacturing, the temperature is desirably 31.1 to 80°C, and is preferably 32 to 60°C.
In this manufacturing, the pressure is desirably equal to or more than 7.38 MPa, and is preferably 7.4 to 50 Mpa.
In this manufacturing, the contact time of supercritical carbon dioxide in the presence of solvent is desirably 0.5 to 20 hours, and is preferably 1 to 5 hours. The contact time of supercritical carbon dioxide is desirably 0.1 to 1 hour, and is preferably 0.1 to 0.5 hour.

### [Manufacturing method 2]

### The manufacture of the VI type crystal

The VI type crystal can be manufactured by contacting the III type crystal or the V type crystal with supercritical carbon dioxide. Especially the III type crystal is an important manufacturing intermediate of the VI type crystal.
In this manufacturing, the temperature is desirably 31.1 to 80°C, and is preferably 32 to 60°C. In this manufacturing, the pressure is desirably equal to or more than 7.38 MPa, and is preferably 7.4 to 50 Mpa.
In this manufacturing, the time is desirably 0.5 to 20 hours, and is preferably 1 to 10 hours.

The crystal of piperacillin sodium of the present invention can be converted to injections according to conventional methods.
Furthermore, the crystal of piperacillin sodium of the present invention can be converted to combination drugs by combining well-known beta-lactamase inhibitors, for example, clavulanic acid, sulbactam and/or tazobactam and the like. For desirable beta-lactamase inhibitors, tazobactam is given, and the combining ratio is not limited in particular, but the crystals of 4 to 8 (a weight ratio) of piperacillin sodium per one tazobactam are desirable.

Next, the V type crystal and the III type crystal which is an important manufacturing intermediate of the VI type crystal are explained. Because the III type crystal has excellent solubility, low hygroscopicity and the powdery bulk is smaller than that of the amorphous body and the electrostatic propensity is small, the handling of the crystal is very easy in the case of the manufacture of a bulk drug and in the case of the filling at formulation. In addition, these crystals are easily filtrated and dried, have low content of the relative substances, and are suitable for a medicinal bulk drug.

Dissolution test, hygroscopic test and purity test were performed by the same testing methods described above to elucidate the utility of the III type crystal. In the dissolution test, the III type crystal dissolved in a short period of time than the amorphous body. In addition, in the hygroscopic test, the III type crystal was superior in hygroscopic stability to the amorphous body. Furthermore, in the purity test, the III type crystal had smaller content of relative substances than the amorphous body.

Next, the manufacturing process of the III type crystal is explained.
Next, the III type crystal can be produced by manufacturing processes, for example, shown in next.

### [Manufacturing method A]

### The manufacture of the III type crystal

The III type crystal can be manufactured by adding a base to the solution of piperacillin monohydrate or the salt thereof.
For solvents used in this manufacture, at least one kind of solvent selected from alkylketones, ethers and alkyl esters, a mixed solvent of N,N-dimethylformamide and water are given.
For alkylketones, acetone, 2-butanone and methyl isobutyl ketone are desirable, and acetone and 2-butanone are preferable.
For ethers, tetrahydrofuran, 1,2-dimethoxyethane, t-butyl methyl ether and 1,4-dioxanes are desirable, and tetrahydrofuran and 1,2-dimethoxyethane are preferable.
For alkyl esters, methyl acetate and ethyl acetate are desirable, and methyl acetate is preferable.
For mixed solvents, at least one kind of solvent selected from alkylketones, ethers and alkyl esters, a mixed solvent of N, N-dimethylformamide and water are desirable, and at least one kind of solvent selected from acetone, 2-butanone, methyl isobutyl ketone, tetrahydrofuran, 1,2-dimethoxyethane, methyl acetate and ethyl acetate, a mixed solvent of N,N-dimethylformamide and water are preferable, and at least one kind of solvent selected from acetone, 2-butanone, tetrahydrofuran and methyl acetate, a mixed solvent of N,N-dimethylformamide and water are furthermore desirable.
The ratio of organic solvent and water is not limited in particular, but, for example, the ratio of the content of organic solvent is desirably 50 to 99 (v/v)%, and is preferably 80 to 99 (v/v)% and is furthermore desirably 95 to 99 (v/v)%.
In addition, the mixing ratio of the organic solvent is not limited in particular, but, for example, in the case of a mixed solvent of alkylketones and N,N-dimethylformamide, the ratio of the content of alkylketones is desirably 60 to 95 (v/v)%, is preferably 70 to 95 (v/v)%, and is furthermore desirably 80 to 95 (v/v)%.
In the case of a mixed solvent of ethers and N,N-dimethylformamide, the ratio of the content of ethers is desirably 60 to 95 (v/v)%, is preferably 70 to 95 (v/v)%, and is furthermore desirably 80 to 95 (v/v)%.
In the case of a mixed solvent of alkyl esters and N,N-dimethylformamide, the ratio of the content of alkyl ester is desirably 60 to 95 (v/v)%, is preferably 70 to 95 (v/v)%, and is furthermore desirably 80 to 95 (v/v)%.

The solution of piperacillin monohydrate or the salt thereof can be prepared by, for example, dissolving piperacillin monohydrate or the salt thereof in at least one kind of solvent selected from alkylketones, ethers and alkyl esters, a mixed solvent of N,N-dimethylformamide and water.
Piperacillin monohydrate can be produced by, for example, methods disclosed in Japanese Patent Laid-Open No. S51-23284 bulletin (patent document 3).
For the salt of piperacillin, for example, sodium salt and triethylamine salt are given.
Piperacillin sodium can be produced by, for example, methods disclosed in Japanese Patent Laid-Open No. S51-23284 bulletin (patent document 3).
The temperature where the solution is prepared is not limited in particular, but is desirably 0 to 30°C, and is preferably 5 to 20°C.

In addition, the solution of piperacillin salt can be prepared by, for example, dissolving piperacillin monohydrate in at least one kind of solvent selected from alkylketones, ethers and alkyl esters, a mixed solvent of N,N-dimethylformamide and water, and by adding reagents such as sodium hydrogen carbonate, 2-ethylhexane acid sodium, sodium acetate, sodium propionate, sodium butyrate and triethylamine to the solution.
The amount of reagents is not limited in particular, but one equivalent amount per piperacillin monohydrate is desirable.
The temperature where the solution of piperacillin salt is prepared is not limited in particular, but is desirably 0 to 30°C, and is preferably 5 to 20°C.

The III type crystal can be manufactured by adding a base to the solution of piperacillin monohydrate or the salt thereof prepared in this way.
For the base, sodium acetate, sodium propionate, sodium butyrate and sodium 2-ethylhexanoate are desirable, and sodium propionate and sodium 2-ethylhexanoate are preferable.
For the addition amount of the base, one equivalent amount is desirable per piperacillin monohydrate.
The temperature of addition and crystallization is not limited in particular, but is desirably 0 to 30°C, and is preferably 5 to 20°C.
The amount of solvent where the crystallization is carried out is not limited in particular, but is desirably 5 to 20 times amount per piperacillin monohydrate or the salt thereof.
In this manufacture, a seed crystal of the III type crystal can be used, and its amount is not limited in particular.

### EXAMPLES

The present invention is explained concretely by listing representative examples below, but these examples do not restrict the present invention at all.
Mixing ratios in fluid mixture are weight ratios.

### Reference example 1

Piperacillin sodium 2.0 g was added to the mixture of 2-butanone 2 mL, N,N-dimethylformamide 3.8 mL and water 0.6 mL, and 2-butanone 10 mL was dropped to the mixture. After insoluble substances were filtrated off, the filtrate was allowed to stand at 5°C for 24 hours. The crystals were filtrated and collected, subsequently washed with a mixture of 2-butanone 3 ml, N,N-dimethylformamide 0.9 mL and water 0.1 mL, 2-butanone 6 mL and 99% acetone 6 mL to give the 1.7 g of crystals of piperacillin sodium monohydrate (the III type crystal). This was allocated to a seed crystal of the III type crystal.
Water content: 3.2%
IR (ATR) 1772, 1715cm⁻¹
The pattern of the powder X-ray diffraction analysis accorded with that of Reference example 2.

### Reference example 2

Piperacillin monohydrate 300 g was added to a mixture of 2-butanone 600 mL, N,N-dimethylformamide 180 mL and water 45 mL.
Sodium propionate 53.8 g was added to this solution at 10 to 20°C. After insoluble substances were filtrated off, -butanone 450 ml was dropped, subsequently the seed crystal of the III type crystal was added, and the mixture was stirred at 15 to 20°C for 2 hours.
Subsequently, 2-butanone 1050 ml was dropped, and the mixture was stirred at 15 to 20°C for 1hour and at 5 to 10°C for 2 hours. The crystals were filtrated and collected, subsequently washed with a mixture of 2-butanone 291 ml, N,N-dimethylformamide 6 ml and water 3 ml, and washed with 2-butanone 300 ml to give the 300 g of crystals of piperacillin sodium monohydrate (the III type crystal).
Water content: 3.3%
IR (ATR) 1771, 1715cm⁻¹
The scanning electron micrograph is shown in FIG. 1, the pattern of the powder X-ray diffraction analysis is shown in FIG. 5 and the infrared absorption spectrum (ATR method) is shown in FIG. 9.

### Example 1

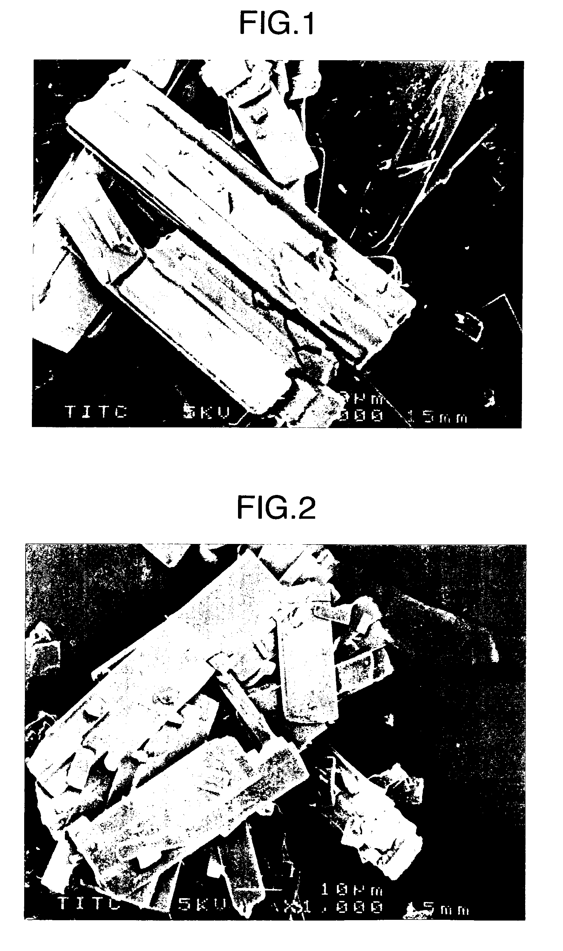

The III type crystal of piperacillin sodium monohydrate 50 g was put in a pressure vessel(68 mm inside diameter, 180 mm length) equipped with a filter, a fluid mixture (carbon dioxide: methyl acetate =96:4) was introduced at 40°C and 20 MPa for 1 hour (flow rate 2.4 kg/h). Subsequently, carbon dioxide was introduced (flow rate 2.4 kg/h) for 0.5 hour at the same temperature and the same pressure. The release of pressure yielded 50 g of crystals of piperacillin sodium monohydrate (the V type crystal).
Water content: 2.8%
IR (ATR) 1772, 1716cm⁻¹
The scanning electron micrograph is shown in FIG. 2, the pattern of the powder X-ray diffraction analysis is shown in FIG. 6 and the infrared absorption spectrum (ATR method) is shown in FIG. 10.

### Example 2

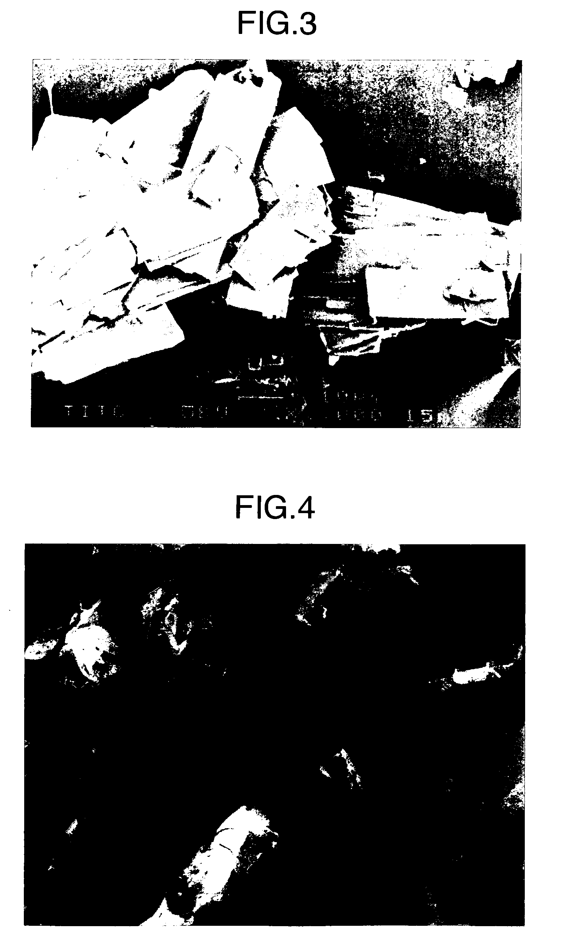

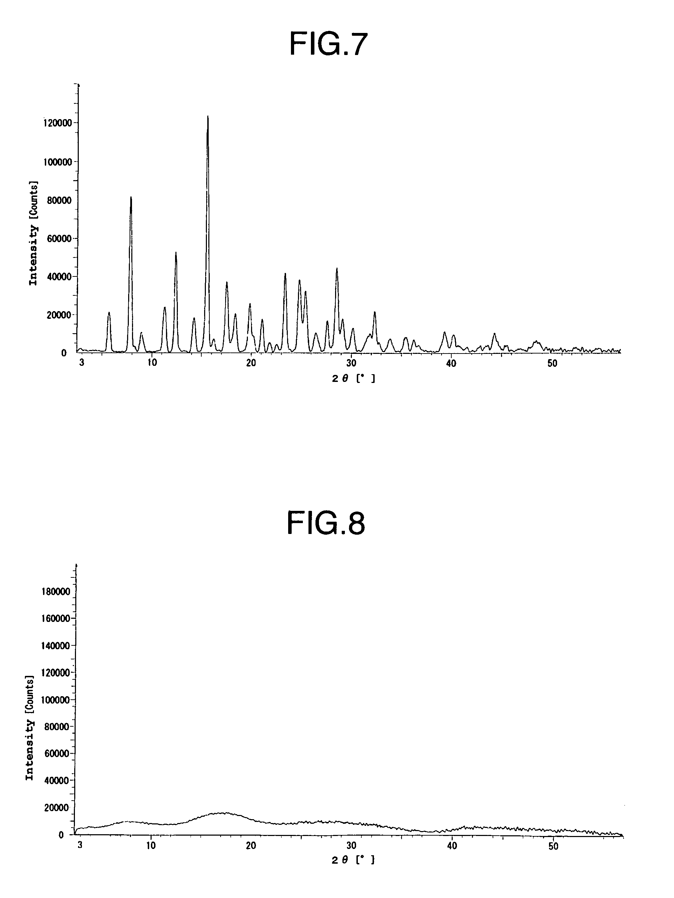

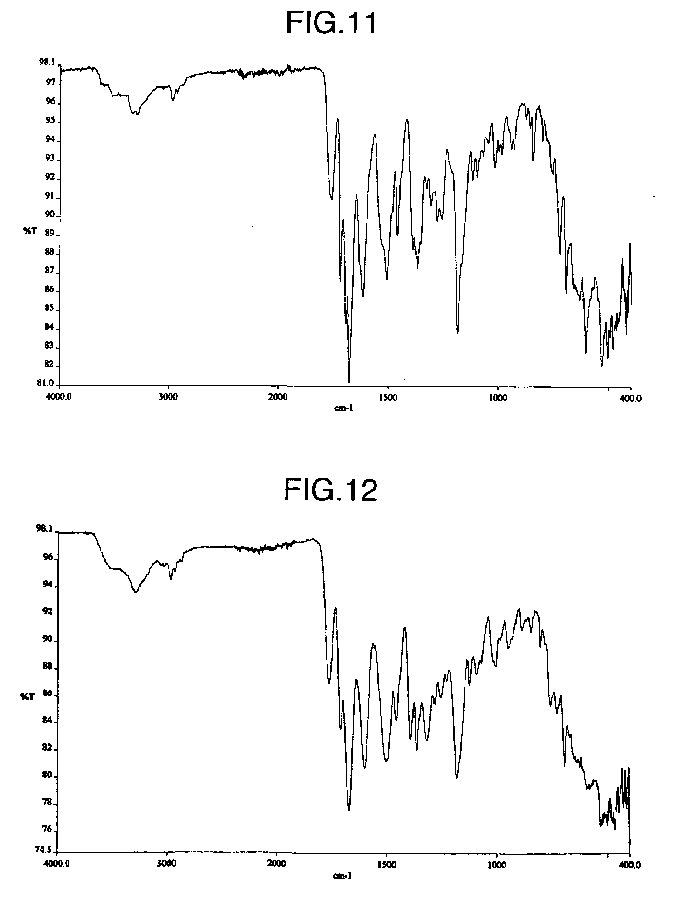

The III type crystal of piperacillin sodium monohydrate 200 g was put in a pressure vessel (68 mm inside diameter, 180 mm length) equipped with a filter, carbon dioxide was introduced at 40°C and 40 MPa for 4 hours (flow rate 2.4 kg/h). The release of pressure yielded 200 g of crystals of piperacillin sodium monohydrate (the VI type crystal).
Water content: 2.8%
IR (ATR) 1764, 1721cm⁻¹
The scanning electron micrograph is shown in FIG. 3, the pattern of the powder X-ray diffraction analysis is shown in FIG. 7 and the infrared absorption spectrum (ATR method) is shown in FIG. 11.

### Example 3

The III type crystal of piperacillin sodium monohydrate 100 g was put in a pressure vessel (68 mm inside diameter, 180 mm length) equipped with a filter, carbon dioxide was introduced at 60°C and 20 MPa for 3 hours (flow rate 2.4 kg/h). The release of pressure yielded 100 g of crystals of piperacillin sodium monohydrate (the VI type crystal).
Water content: 3.1%
IR (ATR) 1764, 1721cm⁻¹
The pattern of the powder X-ray diffraction analysis accorded with that of Example 2.

### Example 4

The V type crystal of piperacillin sodium monohydrate 30 g was put in a pressure vessel (68 mm inside diameter, 180 mm length) equipped with a filter, carbon dioxide was introduced at 40°C and 20 MPa for 3 hours (flow rate 2.4 kg/h). The release of pressure yielded 30 g of crystals of piperacillin sodium monohydrate (the VI type crystal).
Water content: 3.3%
IR (ATR) 1764, 1721cm⁻¹
The pattern of the powder X-ray diffraction analysis accorded with that of Example 2.

### Comparative example 1 (freeze-dry)

The Piperacillin monohydrate 90 g was added to water 180 mL. Sodium hydrogen carbonate 14 g was added at 6 to 10°C for 2 hours. After insoluble substances were filtrated off, freeze-drying yielded 89 g of piperacillin sodium (the amorphous body).
Water content: 0.5%
IR (ATR) 1765, 1713cm⁻¹
The scanning electron micrograph is shown in FIG. 4, the pattern of the powder X-ray diffraction analysis is shown in FIG. 8 and the infrared absorption spectrum (ATR method) is shown in FIG. 12.

### Comparative example 2 (Japanese Patent Laid-Open No.H02-32082 bulletin, the supplementary examination of the example)

Piperacillin monohydrate 1.1 g was dissolved in acetone 30 mL, ethyl acetate 10 mL solution of sodium 2-ethylhexanoate 0.33 g was dropped at room temperature for 10 minutes. The solid was filtrated and collected, subsequently washed with ethyl acetate 20 mL yielded 1.0 g of piperacillin sodium (the amorphous body).
Water content: 0.5%
IR (ATR) 1764, 1714cm⁻¹
The pattern of the powder X-ray diffraction analysis resembled that of Comparative example 1, and clear peaks were not observed.

### Comparative example 3 (The publication of U.S. patent application No. 2003/028016 specification, the supplementary examination of the Example 1)

Piperacillin monohydrate 3.0 g was added to a mixture of acetone 45 ml and methanol 0.45 mL. After water 0.06 mL was added, a mixed solution of acetone 11 mL of sodium 2-ethylhexanoate 0.99 g and water 0.06 mL was dropped for 30 minutes at room temperature. Subsequently, a mixture of acetone 21 mL and water 0.06 mL was dropped, and the mixture was stirred at 0 to 3°C for 2 hours.
The solid was filtrated and collected, subsequently washed with acetone 20 mL yielded 2.0 g of piperacillin sodium (the amorphous body). Water content: 0.5%
IR (ATR) 1764, 1713cm⁻¹
The pattern of the powder X-ray diffraction analysis resembled that of Comparative example 1, and clear peaks were not observed.

### Pharmaceutical preparation example 1

The crystal of piperacillin sodium monohydrate (the V type crystal) obtained in Example 1 was subdivided and packed into glass vials (1 g/vial, 2 g/vial), and the vials were sealed up with a rubber stopper under reduced pressure, furthermore, the vials were rolled up with an aluminum cap to give the injectable pharmaceutical preparations of piperacillin sodium monohydrate.

### Pharmaceutical preparation example 2

The crystal of piperacillin sodium monohydrate (the VI type crystal) obtained in Example 2 was subdivided and packed into glass vials (1 g/vial, 2 g/vial), and the vials were sealed up with a rubber stopper under reduced pressure, furthermore, the vials were rolled up with an aluminum cap to give the injectable pharmaceutical preparations of piperacillin sodium monohydrate.

### Pharmaceutical preparation example 3

The crystal of piperacillin sodium monohydrate (the V type crystal) obtained in Example 1 was subdivided and packed (2 g/bag) into a plastic double bag (a plurilocular container), the bags were sealed up with a heat sealing to give the injectable pharmaceutical preparation kits of piperacillin sodium monohydrate.

### Pharmaceutical preparation example 4

The crystal of piperacillin sodium monohydrate (the VI type crystal) obtained in Example 2 was subdivided and packed (2 g/bag) into a plastic double bag (a plurilocular container), the bags were sealed up with a heat sealing to give the injectable pharmaceutical preparation kits of piperacillin sodium monohydrate.

### Pharmaceutical preparation example 5

The crystal of piperacillin sodium monohydrate (the V type crystal) 4 g obtained in Example 1 and tazobactam sodium 0.5 g were subdivided and packed into glass vials (4.5 g/vial), and the vials were sealed up with a rubber stopper under reduced pressure, furthermore, the vials were rolled up with an aluminum cap to give the injectable pharmaceutical preparations of piperacillin sodium monohydrate and tazobactam sodium.

### Pharmaceutical preparation example 6

The crystal of piperacillin sodium monohydrate (the VI type crystal) 4 g obtained in Example 2 and tazobactam sodium 0.5 g were subdivided and packed into glass vials (4.5 g/vial), and the vials were sealed up with a rubber stopper under reduced pressure, furthermore, the vials were rolled up with an aluminum cap to give the injectable pharmaceutical preparations of piperacillin sodium monohydrate and tazobactam sodium.

### Pharmaceutical preparation example 7

The crystal of piperacillin sodium monohydrate (the V type crystal) 4 g obtained in Example 1 and tazobactam sodium 0.5 g were subdivided and packed into a plastic double bag (a plurilocular container), and the bags were sealed up with a heat sealing to give the injectable pharmaceutical preparation kits of piperacillin sodium monohydrate and tazobactam sodium.

### Pharmaceutical preparation example 8

The crystal of piperacillin sodium monohydrate (the VI type crystal) 4 g obtained in Example 2 and tazobactam sodium 0.5 g were subdivided and packed into a plastic double bag (a plurilocular container), and the bags were sealed up with a heat sealing to give the injectable pharmaceutical preparation kits of piperacillin sodium monohydrate and tazobactam sodium.

### [BRIEF DESCRIPTION OF THE DRAWINGS]

[FIG. 1] is the scanning electron micrograph (amplification 1,000 times) of crystal of piperacillin sodium monohydrate (the III type crystal).
[FIG. 2] is the scanning electron micrograph (amplification 1,000 times) of crystal of piperacillin sodium monohydrate (the V type crystal).
[FIG. 3] is the scanning electron micrograph (amplification 1,000 times) of crystal of piperacillin sodium monohydrate (the VI type crystal).
[FIG. 4] is the scanning electron micrograph (amplification 200 times) of piperacillin sodium (the amorphous body).
[FIG. 5] is the pattern of the powder X-ray diffraction analysis of crystal of piperacillin sodium monohydrate (the III type crystal).
[FIG. 6] is the pattern of the powder X-ray diffraction analysis of crystal of piperacillin sodium monohydrate (the V type crystal).
[FIG. 7] is the pattern of the powder X-ray diffraction analysis of crystal of piperacillin sodium monohydrate (the VI type crystal).
[FIG. 8] is the pattern of the powder X-ray diffraction analysis of piperacillin sodium (the amorphous body).
[FIG. 9] is the infrared absorption spectrum (ATR method) of crystal of piperacillin sodium monohydrate (the III type crystal).
[FIG.10] is the infrared absorption spectrum (ATR method) of crystal of piperacillin sodium monohydrate (the V type crystal).
[FIG. 11] is the infrared absorption spectrum (ATR method) of crystal of piperacillin sodium monohydrate (the VI type crystal).
[FIG. 12] is the infrared absorption spectrum (ATR method) of piperacillin sodium (the amorphous body).

### INDUSTRIAL APPLICABILITY

The novel crystals of piperacillin sodium of the present invention have superior solubility, and the handling of those is very easy in the case of the manufacture of a bulk drug and in the case of the filling at formulation because of low hygroscopicity.
In addition, these crystals are easily filtrated and dried, have small content of relative substances, are high purity and are suitable for a medicinal bulk drug.
Furthermore, these crystals are superior in stability, and the purity depressions and the colorations (degrees of whiteness and yellowness) of exterior appearance of these crystals are smaller than those of the amorphous body.
From these points, the novel crystals of piperacillin sodium of the present invention and the injectable pharmaceutical preparations which were filled with these crystals are useful.

## Claims

1. The crystal of sodium (2S,5R,6R)-6-((2R)-2-((4-ethyl-2,3-dioxopiperazine-1-carbonyl)amino)-2-phenylacetylamino)-3,3-dimethyl-7-oxo-4-thia-1-azabicyclo[3.2.0]heptane-2-carboxylate monohydrate having diffraction angles of 3.7, 5.5, 7.3, 11.6, 14.5 and 18.0° expressed by 2θ in the powder X-ray diffraction analysis.

2. The crystal of sodium (2S,5R,6R)-6-((2R)-2-((4-ethyl-2,3-dioxopiperazine-1-carbonyl)amino)-2-phenylacetylamino)-3,3-dimethyl-7-oxo-4-thia-1-azabicyclo[3.2.0]heptane-2-carboxylate monohydrate having diffraction angles of 5.6, 7.8, 12.3, 15.5, 17.5, 23.3, 24.8 and 28.5° expressed by 2θ in the powder X-ray diffraction analysis.
